# EUROPEAN PATENT SPECIFICATION

(11) **EP 1 579 881 B1**
(45) Date of publication and mention of the grant of the patent: **04.06.2008**
(21) Application number: 04006896.7
(22) Date of filing: 23.03.2004
(51) Int. Cl.: A61M 5/30, A61M 5/20

(54) **Multi-stage propulsion system for an injection device**
Mehrstufiges Vortriebssystem für ein Injektionsgerät
Système multi-étages de propulsion pour un dispositif d'injection

(43) Date of publication of application: 28.09.2005
(73) Proprietor: TecPharma Licensing AG, 3401 Burgdorf (CH)
(72) Inventor: Neracher, Arnold, 1247 Anieres (CH)

(56) References cited:
- WO-A-01/47586
- WO-A-02/49697
- WO-A-20/04006998

## Description

### Background of the Invention

The present invention relates to a multiple pressure stage propulsion system with a source of stored potential energy in the form of a compressed non-gaseous substance. The invention in particular relates to a propulsion system for use in a needle-free transdermal injection device.

In international patent application WO 01/47586 A1, a propulsion system for a needle-free injection device, comprising a primary source of stored potential energy in the form of a compressed liquid, is described. Preferred compressible substances for the primary energy source are chosen from the family of polysiloxanes in view of their high compressibility compared to other liquids or solids. The provision of a compressed liquid or solid as a primary source of potential energy for propelling a liquid to be injected is advantageous over systems using compressed gas or mechanical springs because of the significantly higher energy density that the compressed liquid or solid offers. Springs, for example, requires large dimensions to obtain the required propulsion energy to ensure that a patient's skin is pierced, and a relatively large liquid jet diameter in order to ensure sufficient power of the jet. Systems using compressed gas are limited by the maximum pressure of the gas until a change of state to the liquid form, which defines the maximum pressure of the propulsion system. The risk of explosion of compressed gas systems is also a safety concern.

The propulsion system known from WO 01/47586 A1 comprises the features of the preamble of claim 1.

The propulsion system described in the aforementioned publication further comprises a secondary source of potential energy generating a lower pressure than the primary source. This enables the injection depth to be accurately controlled, in particular to limit the depth of liquid delivery once the skin has been pierced by the initial high pressure jet. This is important in applications requiring intradermal or subcutaneous delivery.

Examples of the secondary source of potential energy include a metal spring, a gaseous substance, or a compressible liquid substance similar to the substance used in the primary energy source. In the latter example, the liquid, which is advantageously a liquid polysiloxane, is compressed at a high pressure in a first container portion and at a lower pressure in a second container portion. In an embodiment, the first container portion is separated from the second container portion by a movable separating wall. In another embodiment, a movable plug portion connected to the drive piston is inserted in a passage between the container portions. Upon actuation of the system, the primary source of energy exerts a high pressure over a certain length of time on the drive piston of the system in order to produce a liquid micro-jet capable of piercing the skin of a patient. Once the pressure of the primary energy source drops to the pressure of the secondary energy source in the first embodiment, or the plug portion of the drive piston disengages from the passage separating the container portions in the second embodiment, the secondary energy source provides most of the energy, at a lower pressure, to drive the piston and complete injection. The lower pressure of the secondary energy source is adapted to ensure that the liquid to be injected does not penetrate the tissue below the required depth, once the high resistance of the outer layer of skin has been overcome.

The use of liquid polysiloxane in both the high and low pressure energy sources is advantageous since it allows the pressure characteristic of the first and second stages to be adjusted within a large range of values. Moreover, it enables the propulsion device to be very compact, safe and relatively inexpensive.

The multi-stage pressure sources based on liquid polysiloxane described in the aforementioned publication WO 01/47586 A1 are however best adapted to single-use disposable injection devices. There is a need to provide a more versatile multi-stage propulsion system, in particular a multi-stage propulsion system that is also adapted for re-usable injection devices. In addition, there is a continuous need to provide a propulsion system that is reliable and compact, yet inexpensive to manufacture.

### Summary of the Invention

Considering the above, an object of the invention is to provide a versatile reusable multi-stage propulsion system, in particular for use in needle-free transdermal injection devices, that is also reliable and compact.

It would also be advantageous to provide a multi-stage propulsion system that is relatively inexpensive to produce, in particular that comprises few parts.

The invention is a propulsion system according to claim 1.

It is a propulsion system suitable for a needle-free transdermal injection device, said propulsion system comprising a container consisting of a first container portion and a second container portion, a releasable pressure seal, a pressure retaining and release means, a pressure generating mechanism and a pressure generating insert, whereas the first container portion defining a first chamber for receiving a primary energy source principally in the form of a first compressible liquid or solid substance under pressure therein for driving the pressure generating insert during an initial phase after actuation of the propulsion system at an initial force, whereas the first container portion is arranged coaxially and consecutively with the second container portion defining a second chamber for receiving a secondary energy source principally in the form of a second compressible liquid or sold substance under pressure therein for driving the pressure generating insert subsequent the initial phase at a force less than the initial force, wherein the pressure generating insert comprises a first insert portion for inserting in the first chamber and a second insert portion received in the second chamber, whereas the first and second insert portions are coaxially related with respect the first and second chamber. Said releasable pressure seal is positioned at an insertion end of the first chamber and sealingly engages the first insert portion when the propulsion system is in the loaded position. The first chamber and the second chamber of the container may communicate with each other when the propulsion system is in the unloaded position and whereas the releasable pressure seal sealingly blocks a passage between the first chamber and the second chamber of the container when the propulsion system is in the loaded position. The second chamber of the container might have a bigger volume then the first chamber of the container. The first and second chamber of the container may be in communication with each other when the propulsion system is in the unloaded position. The first and second chamber of the container may be separate. The second insert portion may extend integrally from the first insert portion. The first and second insert portions might be separate and distinct pressure generating inserts received respectively in the first and second chamber of the container.

In the loaded position, the releasable pressure seal positioned at an insertion end of the first chamber sealingly engages the first insert portion, such that the pressure of the compressible substance in the first chamber is higher than the pressure in the second chamber.

According to the invention, actuation of the pressure generating mechanism acts on the pressure generating insert to advance the first insert portion in the first chamber and the second insert portion in the second chamber to increase the pressure therein in order to load the propulsion system.

Advantageously, a particularly compact and safe re-usable multi-stage propulsion system for a needle free injection device, that can accurately deliver a liquid transdermally to a required depth, is provided.

Further objects and advantageous aspects of the invention will be apparent from the following description, claims and accompanying drawings.

### Brief Description of the Drawings

Fig. 1 is a longitudinal section in perspective of an injection device comprising a propulsion system according to this invention, in a loaded position;
Fig. 1a is a partial sectional view of a front portion of the injection device of figure 1;
Fig. 2 is a longitudinal section of the injection device with the propulsion system in an actuated or unloaded position;
Fig. 2a is a londitudinal section of a front portion of the injection device of figure 2;
Fig. 2b is a londitudinal section of a centre portion of the injection device of figure 2;
Fig. 2c is a londitudinal section of a rear portion of the injection device of figure 2; and
Fig. 3 is a graph illustrating the relationship of propulsion force of the propulsion system of the injection device as a function of the axial displacement of the propulsion system piston.

### Detailed Description of the Invention

Referring to the figures, an injection device comprises a propulsion system 1 and a disposable capsule 3 mountable thereto in a housing 5, for the administration of a liquid 2 contained in the capsule under the skin of a human or animal patient.

The propulsion system comprises a container 4, a pressure retaining and release means 6, a pressure generating mechanism 8 and a pressure generating insert 10.

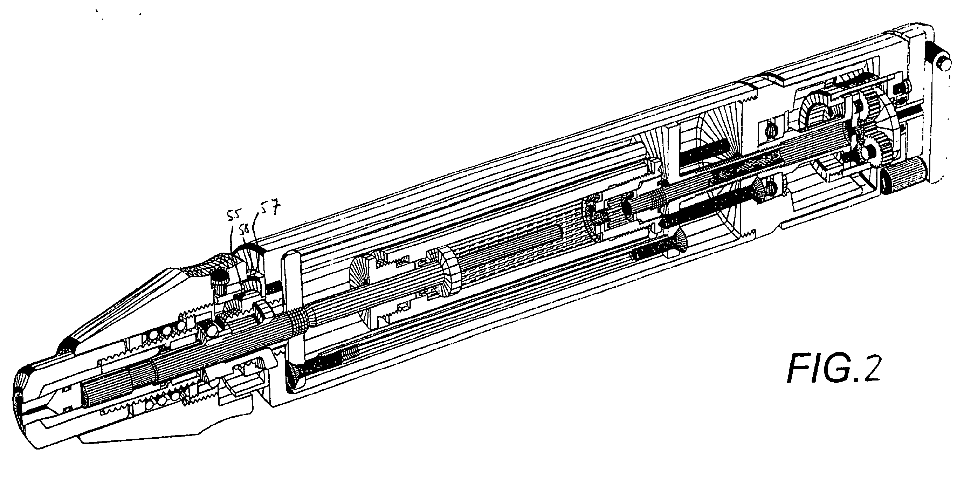

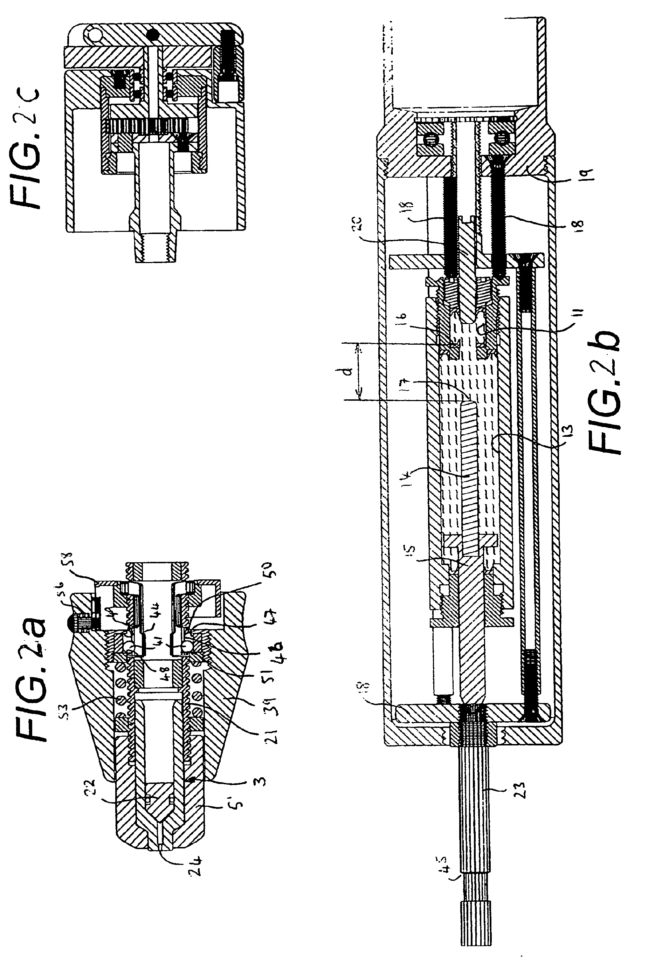

The container 4 comprises a first container portion 9 defining a first (or high pressure) chamber 11 for receiving a first non-gaseous compressible substance 7 therein, and a second container portion 12 defining a second (or low pressure) chamber 13 for receiving a second non-gaseous compressible substance 7' therein. The first and second chambers communicate with each other when the system is in the unloaded position as shown in figure 2.

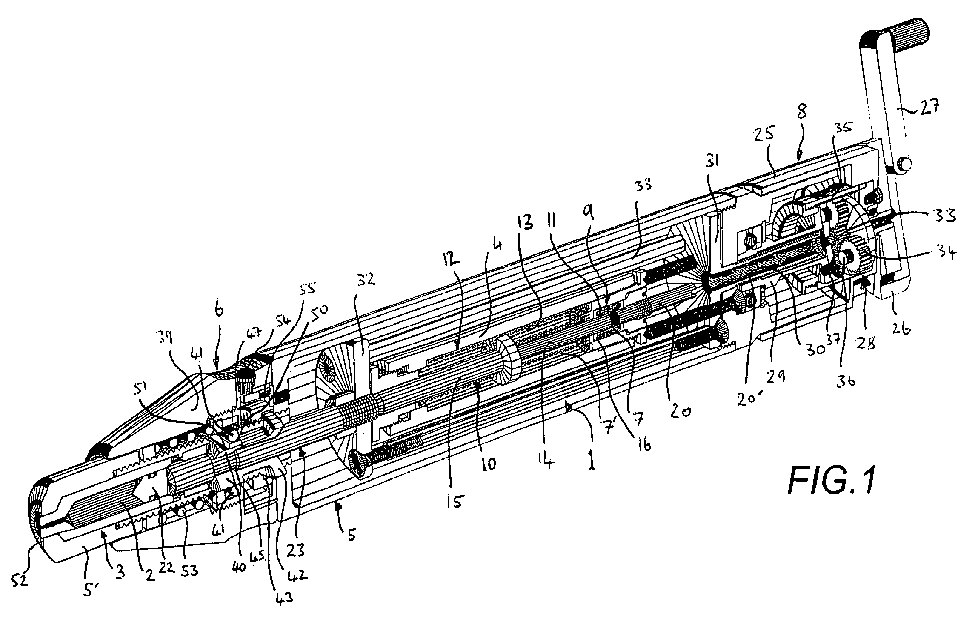

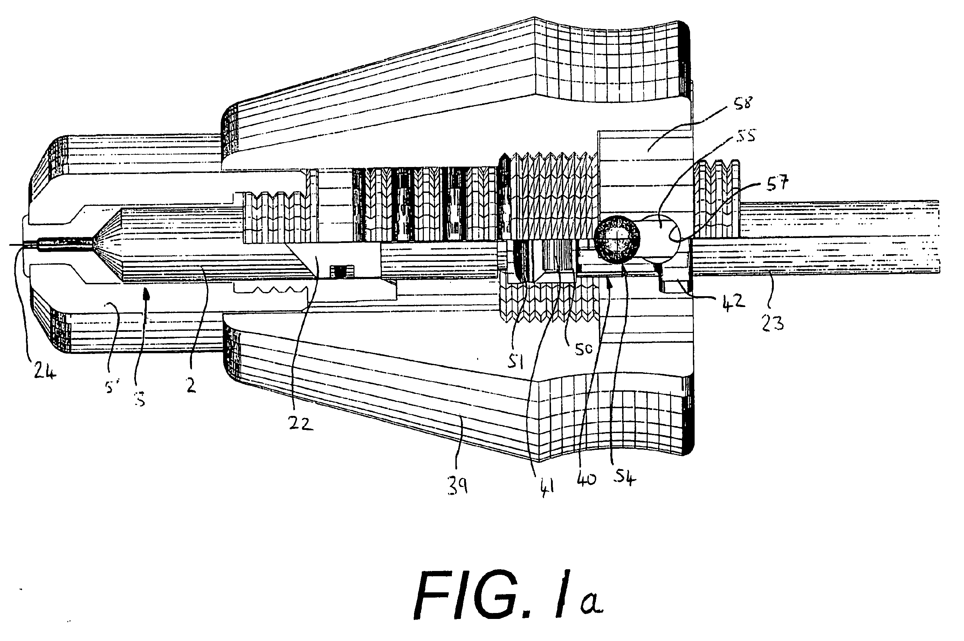

The pressure generating insert comprises a first insert portion 14 for insertion in the first chamber 11, and a second insert portion 15 received in the second chamber 13. A seal 16 positioned at an insertion end of the first chamber sealingly engages the first insert portion 14 when the system is in the loaded position, thus closing the passage between the first and second chambers, as shown in figure 1. As the first insert portion is advanced in the first chamber 11 the pressure therein increases at a faster rate than in the second chamber 13.

This is the result of a greater relative volume change dV/V in the first chamber 11 than in the second chamber 13 as the pressure generating insert advances.

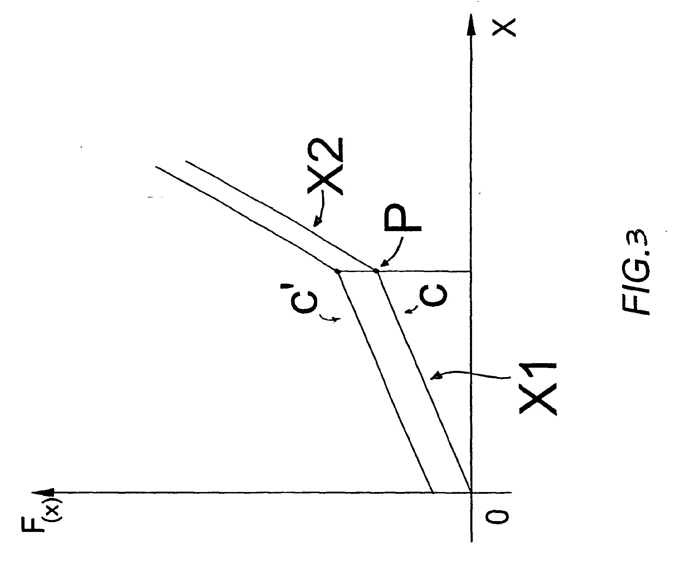

The graph of figure 3 illustrates the force F(x) required to advance the insert 10 as a function of its displacement (x) from the unloaded position (0), the first portion X1 of the curve C having a gradient F(x)/x less than the second portion X2. Point P corresponds to the point at which the free end 17 of the pressure generating insert just enters the first chamber 11 and engages the seal 16 to sealingly block the passage between the first and second chambers. The position of the transition point P between first and second curve portions may be adjusted by defining the distance d in the unloaded state (and thus the displacement) between the free end 17 of the pressure generating insert and the seal 16 of the first chamber. In the present embodiment, this is achieved by varying the length of the screws 18 that are in abutment with a rear end of the container 4 and fixed to a rear wall portion 19 of the injector housing.

The gradients of the portions X1 and X2 can be varied by changing the relative volume change dVN of the first, respectively second chambers, as the pressure generating insert is displaced. In practice, adjusting the respective volumes V1, V2 of the first and second chambers and/or the respective diameters of the first and second insert portions in the design of the propulsion system will enable the relative volume change as a function of displacement to be adjusted in order to produce the optimal pressure characteristic. The adjustable insert 20 can be threaded in or out of the first chamber 11 to change the volume V1 thereof. A central passage 20' through the rear end of the injector provides access for a screwdriver to turn the adjustable insert 20.

Thus, the secondary source of potential energy generates a lower pressure P2 than the maximum pressure P1 generated by the primary potential energy source. The compressed substance of the primary source liberates energy in an initial phase of high pressure injection, peaking for example at around 800 to 1000 bars, followed by liberation of pressure from the secondary source at relatively low pressure, for example 200 bars and less. This double injection pressure stage is very advantageous since it enables the injection depth to be accurately controlled, for example to deliver liquids such as insulin or other medicines intradermally or subcutaneously. The initial high pressure enables a very fine supersonic liquid jet to be formed to pierce skin, followed by the lower pressure second stage jet to deliver the liquid at a controlled depth below the outer surface of the skin, avoiding excessive penetration that would ensue if the initial high pressure were maintained over a longer period.

The double stage propulsion system advantageously enables the desired depth of injection and the volume of injected liquid to be reliably performed by an appropriate selection and design of the primary and secondary potential energy sources, and in particular by adjusting the relative stored energy of each source. Depending on the contribution of the primary energy source relative to the secondary energy source, different injection pressure characteristics over time can be obtained.

The first compressible substance 7 under pressure in the first chamber forms a primary source of potential energy for propelling the liquid to be injected in an initial injection phase. The second compressible substance 7' under pressure in the second chamber forms a secondary source of potential energy for propelling the liquid to be injected in a subsequent injection phase.

The first and second compressible substances may be the same, but may also be different, for example the first substance 7 may be a compressible solid such as vulcanised silicon rubber, and the second substance 7' a liquid polysiloxane. The compressible substance or substances 7,7' may advantageously comprise a polysiloxane oil which has the ability to store a large amount of potential energy through elastic molecular compression, for example up to 100 times more energy than a conventional metal spring occupying the same volume. The elastic property of polysiloxanes is particularly advantageous to the present invention since it allows the injection device to be compact, cost-effective, and comprise few components. Depending on the molecular weight, polysiloxanes typically have volumetric compressibility values (dV/V at a given pressure) three to four times greater that the volumetric compressibility of water. While polysiloxanes are a preferred soft matter for use in the present invention, other liquid substances may also be used.

Polysiloxane oils are relatively inexpensive visco-elastic liquids resistant to high and low temperature, and non-toxic from the physiological point of view, so that they may be used in dermatological and cosmetic applications. Polysiloxane oils have a low viscosity variation as a function of pressure which advantageously facilitates fluid exchange, but they have a high surface tension such that they are non-miscible with water solutions. Polysiloxane oils also have lubricating properties between metals and polymers and rubber, which advantageously facilitates sliding between relative mobile members.

The family of polysiloxane oils comprises, inter alia, the following substances:
- polymethylhydrogensiloxane
- polydimethylsiloxane
- polytrimethylsiloxane
- hexamethylcyclotrisiloxane
- decamethyltetrasiloxane
- hexamethyldisiloxane (H 7310 - Witheco)
- octamethyltrisiloxan (O 9816 - Witheco)
- alpha, beta, gamma, and theta gels from Geltec Corporation.

The front end of the injector housing is provided with a removable cap 5' engaging a threaded portion 21 for releasably mounting the capsule 3 containing the liquid to be injected in the injector housing. Other releasable fixing means could however be provided, such as a bayonet type connection or releasable spring latches. A rear end of the capsule is sealingly closed by a sealing piston 22 that is driven by the propulsion system piston 23 on actuation of the device thereby propulsing the liquid 2 through the capsule nozzle orifice 24. The capsule sealing piston 22 may be provided at its front end with a cone shaped elastic portion in order to ensure that substantially all the liquid to be injected is propelled out of the capsule.

The pressure generating mechanism 8 is mounted to the rear end of propulsion system and comprises a first grip portion 25, a second grip portion 26 with a foldable lever portion 27 and a planetary gear mechanism 28 driving a threaded drive tube 29 engaging a complementary threaded portion 30 of a rear flange 31 fixed to a front flange 32 via rods 33. The front flange is mounted to the propulsion system piston 23. As the first grip portion 25 is turned, the threaded drive tube 29 turns and drives the front flange 31 axially rearwards, thus displacing the insert 10 into the container 4 of the propulsion system and compressing the compressible substance therein. The amount of turns applied to the first grip portion 25 determines the pressure of the compressible substance 7' which can thus be adjusted according to the application.

The second grip portion 26 and foldable lever 27 is fixed to a central pinion 33 that engages satellite gear wheels 34 that in turn engage an outer annular crown gear 35 integral with or fixed to the first grip portion 25. The axes 26 of the satellite gears 34 are mounted pivotly on a flange 37 rigidly fixed to the threaded drive tube 29. In the example illustrated in the figures, the step down gearing ratio is approximately 4:1, i.e. an operator must turn the lever portion approximately 4 full turns for the threaded drive tube 29 to effect one full turn. In use, a human operator may initially turn the first grip portion 25 to compress the propulsion system partially until the torque is too high for the operator, whereby the lever portion 27 may then be folded out, as shown in figure 1, and turned to complete compression of the propulsion system. In this manner, the propulsion system can be quickly loaded by a human operator, while enabling a high final compression level to be attained in the propulsion system.

The pressure generating means may also be driven by an electric motor (not shown) coupled to the central pinion 33. The motor may either be permanently mounted or integrated into the rear end of the injection device, or be provided as a separate unit that removably engages the central pinion 33, for example much like an electric screw driver.

The pressure retaining and release means 6 comprises an actuator member 39, one or more piston retaining elements 40, and one or more blocking inserts 41 mounted between the actuator member and the piston retaining elements.

The retaining elements 40 are pivotly mounted and axially retained in the injector housing by a wall portion 42 thereof extending radially outwards at a rear end of the retaining element and inserted with some play in a corresponding cavity 43 in the housing. The retaining elements however could also be attached to the housing with an elastic hinge to allow the retaining elements to pivot in order to release the propulsion system piston 23. In this example, the two opposed retaining elements are in the form of angular segments, for example roughly 90° segments, of a tubular part. Thus, two pairs of segments can be produced by two perpendicular cuts through the axis of the initial tubular part. The retaining element comprises an inwardly projecting retaining shoulder 44 at a front end thereof engageable with a complementary shoulder 45 of the propulsion system piston 23, in order to retain the propulsion system piston in the loaded position prior to actuation.

The blocking insert 41 is postioned between an outer camming surface 46 of the retaining element and an inner camming surface 47 of the actuator member 39. The inner camming surface 47 is bounded at front and rear ends thereof by front and rear ledges 48, 49 respectively that limit the relative axial displacement of the blocking insert 41. The camming surface of the retaining element 40 has a high point locking surface portion 50 and a low point release surface portion 51. In the loaded position, as shown in figures 1 and 1a, the blocking insert 41 is positioned between the high point locking surface portion 50 and the inner camming surface 47 of the actuator member 39 thereby blocking outward pivoting of the retaining element 40 such that the retaining shoulder thereof remains in engagement with the complementary shoulder 45 of the propulsion system piston 23. The injection device is actuated by displacing the actuator member 39 axially toward the front or injection end 50 of the device, whereby the blocking insert 41 rolls along the retaining element camming surface to the low point release surface portion 47 as shown in figure 2a, such that the retaining elements pivot radially outwards and release the propulsion system piston 23. It may be noted that the retaining elements will tend to pivot outwards about their pivot attachment ends 42 in view of the moment created by the force exerted by the propulsion system piston 23 on the retaining shoulders 44 of the retaining elements 40.

The blocking insert 37 is advantageously an element that rolls along the camming surface in order to reduce frictional force, particularly in view of the high axial force exerted by the propulsion system. The blocking element may be in the form of a solid cylinder, but could also be in the form of a plurality of balls or of a cylinder formed of a spring wire coil.

The pressure retaining and release means may further comprise a return spring 53 that axially biases the actuator member and, as a consequence the blocking insert, into the retaining position shown in figure 1, when the propulsion system piston is drawn back after actuation for reloading. A safety lock in the form of a button 54 for example, may be provided to axially lock the actuator member 39 and prevent indavertent axial displacement of the actuator member. The safety lock may also serve to indicate to a human operator when the injection device is in the loaded position or has been actuated. In this example, the safety lock comprises a locking key portion 55 biased by means of a spring 56 into a complementary key cavity in a cylindrical wall portion 58 of the injection device housing. In the retaining or loaded position as shown in figure 1, the locking key portion 55 engages in the complementary key cavity 57 and axially locks the actuator member 39 and the button 54 protrudes slightly beyond the outer surface of the actuator member 39. In the unlocked and actuated positions, the button 54 is in a depressed position below the outer surface as seen in figure 2, and the key portion 55 is disengaged from the complementary locking cavity 57.

## Claims

1. A propulsion system (1) suitable for a needle-free transdermal injection device, said propulsion system comprising a container (4) consisting of a first container portion (9) and a second container portion (12), a releasable pressure seal (16), a pressure retaining and release means (6), and a pressure generating insert (10), whereas the first container portion (9) defining a first chamber (11) containing a primary energy source principally in the form of a first compressible non-gaseous substance(7) under pressure therein for driving the pressure generating insert (10) during an initial phase after actuation of the propulsion system at an initial force, whereas the first container portion (9) is arranged coaxially and consecutively with the second container portion (12) defining a second chamber (13) containing a secondary energy source principally in the form of a second compressible non-gaseous substance (7') under pressure therein for driving the pressure generating insert (10) subsequent the initial phase at a force less than the initial force, wherein the pressure generating insert (10) comprises a first insert portion (14) for Inserting in the first chamber (11) and a second insert portion (15) received in the second chamber (13), whereas the first and second insert portions (14,15) are coaxially related with respect the first and second chamber (11,13), said releasable pressure seal (16) positioned at an insertion end of the first chamber (11) sealingly engages the first insert portion (14) when the propulsion system (1) is in the loaded position, **characterised in that** the system comprising a pressure generating mechanism (8) and actuation of the pressure generating mechanism acts on the pressure generating insert (10) to advance the first insert portion in the first chamber and the second insert portion in the second chamber to increase the pressure therein in order to load the propulsion system.

2. A propulsion system (1) according to the preceding claim wherein the propulsion system (1) is a reloadable propulsion system whereas the first chamber (11) and the second chamber (13) of the container communicate with each other when the propulsion system is in the unloaded position and whereas the releasable pressure seal (16) sealingly blocks a passage between the first chamber (11) and the second chamber (13) of the container when the propulsion system (1) is in the loaded position.

3. The propulsion system (1) according to claim 1 wherein the second chamber (13) of the container having a bigger volume then the first chamber (11) of the container.

4. The propulsion system (1) according to claim 1,2 or 3 wherein the first and second chamber (11, 13) of the container are in communication with each other when the propulsion system (1) is in the unloaded position.

5. The propulsion system (1) according to claim 1, 2 or 3 wherein the first and second chamber (11,13) of the container are separate.

6. The propulsion system (1) according to claim 4 wherein the second insert portion (15) extends integrally from the first insert portion (14).

7. The propulsion system (1) according to claim 5 wherein the first and second insert portions (14,15) are separate and distinct pressure generating inserts (10) received respectively in the first and second chamber (11,13) of the container.

## Patentansprüche

1. Vortriebssystem (1), das für eine nadelfreie transdermale Injektionsvorrichtung geeignet ist, wobei das Vortriebssystem einen Behälter (4), der aus einem ersten Behälterabschnitt (9) und einem zweiten Behälterabschnitt (12) besteht, eine lösbare Druckdichtung (16), ein Druckhalte- und Druckfreigabemittel (6) und einen Druckerzeugungseinsatz (10) umfasst, wobei der erste Behälterabschnitt (9) eine erste Kammer (11) definiert, die eine primäre Energiequelle hauptsächlich in Form einer ersten komprimierbaren nicht gasförmigen Substanz (7) enthält, die darin unter Druck steht, um den Druckerzeugungseinsatz (10) während einer Anfangsphase nach der Betätigung des Vortriebssystems mit einer Anfangskraft anzutreiben, wobei der erste Behälterabschnitt (9) koaxial zu dem zweiten Behälterabschnitt (12) und diesem folgend angeordnet ist, wobei der zweite Behälterabschnitt (12) eine zweite Kammer (13) definiert, die eine zweite Energiequelle hauptsächlich einer zweiten komprimierbaren nicht gasförmigen Substanz (7') enthält, die darin unter Druck steht, um den Druckerzeugungseinsatz (10) nach der Anfangsphase mit einer Kraft anzutreiben, die kleiner als die Anfangskraft ist, wobei der Druckerzeugungseinsatz (10) einen ersten Einsatzabschnitt (14), zum Einsetzen in die erste Kammer (11), und einen zweiten Einsatzabschnitt (15), der in der zweiten Kammer (13) aufgenommen ist, umfasst, wobei der erste und der zweite Einsatzabschnitt (14, 15) in Bezug auf die erste und die zweite Kammer (11, 13) in einer koaxialen Beziehung stehen, und die lösbare Druckdichtung (16), die an einem Einsetzende der ersten Kammer (11) positioniert ist, mit dem ersten Einsatzabschnitt (14) in einen dichten Eingriff gelangt, wenn sich das Vortriebssystem (1) in der geladenen Position befindet, **dadurch gekennzeichnet, dass** das System einen Druckerzeugungsmechanismus (8) umfasst und eine Betätigung des Druckerzeugungsmechanismus auf den Druckerzeugungseinsatz (10) wirkt, um den ersten Einsatzabschnitt in der ersten Kammer und den zweiten Einsatzabschnitt in der zweiten Kammer vorwärts zu bewegen, um den Druck darin zu erhöhen, um das Vortriebssystem zu laden.

2. Vortriebssystem (1) nach dem vorhergehenden Anspruch, wobei das Vortriebssystem (1) ein wieder ladbares Vortriebssystem ist, wobei die erste Kammer (11) und die zweite Kammer (13) des Behälters miteinander kommunizieren, wenn das Vortriebssystem in der nicht geladenen Position ist, und wobei die lösbare Druckdichtung (16) einen Durchgang zwischen der ersten Kammer (11) und der zweiten Kammer (13) des Behälters abdichtend blockiert, wenn sich das Vortriebssystem (1) in der geladenen Position befindet.

3. Vortriebssystem (1) nach Anspruch 1, wobei die zweite Kammer (13) des Behälters ein größeres Volumen als die erste Kammer (11) des Behälters hat.

4. Vortriebssystem (1) nach Anspruch 1, 2 oder 3, wobei die erste und die zweite Kammer (11, 13) des Behälters miteinander kommunizieren, wenn sich das Vortriebssystem (1) in der nicht geladenen Position befindet.

5. Vortriebssystem (1) nach Anspruch 1, 2 oder 3, wobei die erste und die zweite Kammer (11, 13) des Behälters voneinander getrennt sind.

6. Vortriebssystem (1) nach Anspruch 4, wobei sich der zweite Einsatzabschnitt (15) von dem ersten Einsatzabschnitt (14) integral erstreckt.

7. Vortriebssystem (1) nach Anspruch 5, wobei der erste und der zweite Einsatzabschnitt (14, 15) getrennte und verschiedene Druckerzeugungseinsätze (10) sind, die in der ersten bzw. in der zweiten Kammer (11, 13) des Behälters aufgenommen sind.

## Revendications

1. Système de propulsion (1) approprié pour un dispositif d'injection transdermique sans aiguille, ledit système de propulsion comprenant un réservoir (4) consistant en une première portion de réservoir (9) et en une seconde portion de réservoir (12), un joint d'étanchéité libérable à la pression (16), des moyens de libération et de retenue à la pression (6) et un insert générant la pression (10), tandis que la première portion de réservoir (9) définissant une première chambre (11) contenant une source d'énergie primaire, principalement sous forme d'une première substance non gazeuse compressible (7) sous pression pour entraîner l'insert générant la pression (10) pendant une phase initiale après l'actionnement du système de propulsion à une force initiale, tandis que la première portion de réservoir (9) est disposée coaxialement et consécutivement à la seconde portion de réservoir (12) définissant une seconde chambre (13) contenant une source d'énergie secondaire principalement sous forme d'une seconde substance non gazeuse compressible (7') sous pression pour entraîner l'insert générant la pression (10) consécutivement à la phase initiale à une force inférieure à la force initiale, dans lequel l'insert générant la pression (10) comprend une première portion d'insert (14) pour l'insertion dans la première chambre (11) et une seconde portion d'insert (15) reçue dans la seconde chambre (13), tandis que les première et seconde portions d'insert (14, 15) sont situées coaxialement par rapport à la première et à la seconde chambre (11, 13), ledit joint d'étanchéité libérable à la pression (16), positionné à une extrémité d'insertion de la première chambre (11), coopère de façon étanche avec la première portion d'insert (14) lorsque le système de propulsion (1) est dans la portion chargée, **caractérisé en ce que** le système comprenant un mécanisme générant la pression (8) et l'actionnement du mécanisme générant la pression agit sur l'insert générant la pression (10) pour faire avancer la première portion d'insert dans la première chambre et la seconde portion d'insert dans la seconde chambre pour y augmenter la pression, afin de charger le système de propulsion.

2. Système de propulsion (1) selon la revendication précédente, dans lequel le système de propulsion (1) est un système de propulsion rechargeable, moyennant quoi la première chambre (11) et la seconde chambre (13) du réservoir communiquent entre elles lorsque le système de propulsion est dans la position non chargée et moyennant quoi le joint d'étanchéité à pression libérable (16) bloque de façon étanche un passage entre la première chambre (11) et la seconde chambre (13) du réservoir lorsque le système de propulsion (1) se trouve dans la position chargée.

3. Système de propulsion (1) selon la revendication 1, dans lequel la seconde chambre (13) du réservoir présente un plus grand volume que la première chambre (11) du réservoir.

4. Système de propulsion (1) selon la revendication 1, 2 ou 3, dans lequel les première et seconde chambres (11, 13) du réservoir sont en communication entre elles lorsque le système de propulsion (1) est dans la position non chargée.

5. Système de propulsion (1) selon la revendication 1, 2 ou 3, dans lequel les première et seconde chambres (11, 13) du réservoir sont séparées.

6. Système de propulsion (1) selon la revendication 4, dans lequel la seconde portion d'insert (15) s'étend solidairement à partir de la première portion d'insert (14).

7. Système de propulsion (1) selon la revendication 5, dans lequel les première et seconde portions d'insert (14, 15) sont des inserts générant la pression, séparés et distincts (10), logés respectivement dans les première et seconde chambres (11, 13) du réservoir.
